# EUROPÄISCHE PATENTSCHRIFT

(11) **EP 1 603 927 B1**
(45) Veröffentlichungstag und Bekanntmachung des Hinweises auf die Patenterteilung: **20.08.2008**
(21) Anmeldenummer: 04714689.9
(22) Anmeldetag: 26.02.2004
(51) Int. Cl.: C07F 9/38, A61K 31/663, A61P 3/06

(54) **OPTISCH DETEKTIERBARE (ALKYLACYL-2-AMINODESOXY-GLYCERO) -P- NITROPHENYL-PHOSPHONATE**
OPTICALLY DETECTABLE (ALKYLACYL-2-AMINODESOXY-GLYCERO)-NITROPHENYL-PHOSPHONATES
(ALKYLACYL-2-AMINODESOXY-GLYCERO)-P-NITROPHENYL-PHOSPHONATES DETECTABLES PAR VOIE OPTIQUE

(30) Priorität: 19.03.2003 AT 4402003
(43) Veröffentlichungstag der Anmeldung: 14.12.2005
(73) Patentinhaber: Austria Wirtschaftsservice Gesellschaft mit beschränkter Haftung, 1030 Wien (AT); Hermetter, Albin, 8010 Graz (AT)
(72) Erfinder: HERMETTER, Albin, A-8010 Graz (AT); OSKOLKOVA, Olga, A-1040 Wien (AT)
(74) Vertreter: Schwarz, Albin
(86) Internationale Anmeldenummer: PCT/AT2004/000058
(87) Internationale Veröffentlichungsnummer: WO 2004/083220

(56) Entgegenhaltungen:
- ZANDONELLA G ET AL: "INTERACTIONS OF FLUORESCENT TRIACYLGLYCEROL ANALOGS COVALENTLY BOUND TO THE ACTIVE SITE OF A LIPASE FROM PHIZOPUS ORYZAE" EUROPEAN JOURNAL OF BIOCHEMISTRY, BERLIN, DE, Bd. 262, Nr. 1, Mai 1999 (1999-05), Seiten 63-69, XP001023841 ISSN: 0014-2956

## Beschreibung

Die vorliegende Erfindung betrifft optisch detektierbare Organophosphonate. Insbesondere bezieht sie sich auf fluoreszierende Organophosphonate, welche Lipaseinhibitoren sind.

Fettspaltende Enzyme sind in Forschung und Industrie weithin verwendete Biokatalysatoren für die Erzielung chemischer Reaktionen mit hoher Regio- und Stereoselektivität, die enantiomere Alkohole oder Amine erbringen (Jaerger et al., Trends Biotech. 16, 396-403 (1998); Schmid R. und Verger, R.D. Angew. Chem. 110, 1694-1720 (1998); Schmid et al., Nature 409, 258-268 (2001); Koeller et al., Nature 409, 232-240 (2001); Klibanov, Nature 409, 241-246 (2001)).

Lipasen katalysieren die Hydrolyse und Synthese von Triacylglycerinen. Alle Lipasen akzeptieren die Ester mittel- (C4) und langkettiger (C16) gesättigter Fettsäuren als Substrate, und zwar hauptsächlich in *sn-1-* oder sn-3-Positionen (Rangheard et al., Enzyme Microb. Technol. 14, 966-974 (1992); Kirk et al., Biocatalysis 6,127-134 (1992); Ransac et al., J. Biol. Chem. 265, 20263-20270 (1990); Rogalska et al., Chirality 5, 24-30 (1993)).

Der Wirkungsmechanismus dieser Enzyme umfasst die nucleophile Spaltung einer Esterbindung durch ein aktiviertes Serin, das zur katalytischen Triade Ser-His-Asp/Glu gehört (Cygler et al., Methods Enzymol. 284, 3-27 (1997); Jaeger et al., siehe oben; Schmid, R. und Verger, R.D., siehe oben).

Lipophile p-Nitrophenylphosphonatester sind geeignete Hilfsmittel in der Lipaseforschung; z.B. für Studien über Substrat-Enzym-Wechselwirkungen auf molekularer Ebene (Ransac et al., Methods Enzymol. 186,190-231 (1997)) sowie für eine Funktionsanalyse, z.B. zwecks Bestimmung der aktiven Enzymfraktion von rohen oder reinen Proteinzubereitungen (Scholze et al., Analyt. Biochem. 276, 72-80 (1999); Rotticci et al., Biochim. Biophys. Acta 1483, 132-140 (2000)).

Diese Inhibitoren reagieren mit dem nucleophilen Serin von Lipasen, was zur Bildung kovalenter und äquimolarer Lipid-Protein-Komplexe führt, die in wässrigen und organischen Lösungen stabil sind (Rotticci et al., siehe oben; Ransac et al., 1997, siehe oben; Bjoerkling et al., Biorgan. Med. Chem. 2, 697-705 (1994); Zandonella et al., Eur. J. Biochem. 262, 63-69 (1999)). Solche Komplexe stellen "offene" Lipasestrukturen dar und imitieren die Substrat-Enzym-Wechselwirkungen im ersten (tetraedrischen) Übergangszustand.

Fluoreszierende Markierungen im hydrophoben Schweif der Organophosphonate sind nicht nur nützlich für eine quantitative Analyse von Lipasen, sondern auch für die Untersuchung der Lipid-Protein-Wechselwirkungen im ersten Übergangszustand bei unterschiedlichen Umwelt- (Lösungsmittel-) Bedingungen (Oskolkova, O.V. und Hermetter, A., Biochim. Biophys. Acta 1597, 60-66 (2002); Zandonella et al., siehe oben).

Es besteht jedoch ein Bedarf an weiteren Verbindungen für analytische und mechanistische Studien über fettspaltende Enzyme.

Demgemäß besteht das Ziel der vorliegenden Erfindung in der Schaffung von Verbindungen, die als Inhibitoren von fettspaltenden Enzymen nützlich sind. Insbesondere sollten diese Verbindungen nützliche Hilfsmittel für die Analyse sowie die Unterscheidung fettspaltender Enzyme in biologischen Proben darstellen.

Dieses Ziel wird erreicht durch Verbindungen mit der allgemeinen Formel I 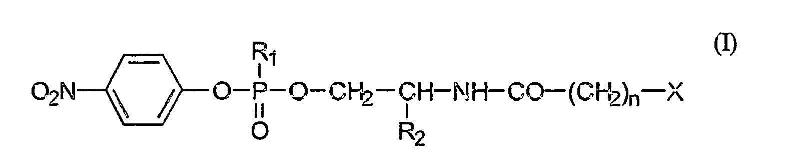 wobei X einen optisch detektierbaren Rest darstellt, n eine ganze Zahl ist, wobei 1 ≤ n ≤ 20, R₁ eine unverzweigte oder verzweigte Alkylgruppe mit 1 bis 20 Kohlenstoffatomen und R₂ Wasserstoff oder eine -CH₂-O-R₃-Gruppe ist, wobei R₃ dieselbe Bedeutung hat wie R₁.

Vorzugsweise ist n eine ganze Zahl, wobei 3 ≤ n ≤ 11.

Bei einer bevorzugten Ausführungsform ist R₁ Hexyl und R₂ eine -CH₂-O-R₃-Gruppe, wobei R₃ Octyl oder Hexadecyl ist.

Gemäß einer weiteren bevorzugten Ausführungsform ist R₁ Methyl und R₂ eine -CH₂-O-R₃-Gruppe, wobei R₃ Octyl oder Hexadecyl ist.

Gemäß einer anderen bevorzugten Ausführungsform ist R₁ Butyl und R₂ eine -CH₂-O-R₃-Gruppe, wobei R₃ Octyl oder Hexadecyl ist.

Bei einer weiteren bevorzugten Ausführungsform ist der optisch detektierbare Rest ein Fluorophor, vorzugsweise eine Perylen-, Pyren- oder Nitrobenzoxadiazol (NBD)-Gruppe.

Gemäß einem weiteren Aspekt bezieht sich die vorliegende Erfindung auf die Verwendung der neuen Verbindungen zur Hemmung fettspaltender Enzyme. Sie bezieht sich weiters auf die Verwendung der neuen Verbindungen für die Bestimmung und/oder Unterscheidung fettspaltender Enzyme in biologischen Proben.

Es wurde bereits berichtet, dass Di-O-alkylglycerophosphonate nützliche Inhibitoren der Lipaseaktivität sind (Stadler et al., Biochim. Biophys. Acta 1304, 229-244 (1996); Zandonella et al., siehe oben). Es gab jedoch keinen Anhaltspunkt dafür, dass mikrobielle Lipasen mit fluoreszierenden Phosphonatinhibitoren, die Amidbindungen enthalten, reagieren würden.

Es wurde herausgefunden, dass die erfindungsgemäßen Verbindungen mikrobielle Lipasen hemmen können. Überdies wurde herausgefunden, dass die erfindungsgemäßen Verbindungen unterschiedliche hemmende Wirkungen auf die Alctivität verschiedener Lipasen aufweisen, wodurch eine Unterscheidung der fettspaltenden Enzyme ermöglicht wird.

Die Erfindung wird nunmehr anhand der nachfolgenden Beispiele und Figuren detaillierter beschrieben, wobei
Fig. 1 die Syntheserouten fluoreszenzmarkierter erfindungsgemäßer Organophosphonate veranschaulicht;
Fig. 2 die Synthese erfindungsgemäßer einkettige Perylen- und NBD (Nitrobenzoxadiazol)-Inhibitoren veranschaulicht; und
Fig. 3a bis 3c im Blockdiagramm die Hemmung mikrobieller Lipasen von *Rhizopus oryzae* (ROL), *Pseudomonas cepacia* (PCL) bzw. *Pseudomonas species* (PSL) darstellen.

### Beispiele

### Materialien und Verfahren:

Die Standardchemikalien wurden von Merck bezogen. Mercaptoethanol und *p*-Nitrophenol kamen von Sigma; Methylphosphonsäuredichlorid, *n*-Hexylphosphonsäuredichlorid und Methylsebacoylchlorid kamen von Aldrich. 1-Methylimidazol, Tetrazol (3.5%ige Lösung in Acetonitril, von Sigma-Aldrich), Perylen, 3-Carbomethoxypropionylchlorid und N-Hydroxysuccinimid wurden von Fluka bezogen. Succinimidyl-6-[N-(7-nitrobenz-2-oxa-1,3-diazol-4-yl)amino]hexanoat wurde bei Molecular Probes erworben .und 12-[N-(7-Nitrobenz-2-oxa-1,3-diazol-4-yl)amino]dodecansäure bei Lambda Fluoreszenztechnologie GmbH (Graz, Österreich).

Aktivierte Succinimidester von fluoreszenzmarkierten Säuren wurden aus geeigneten Fettsäuren und N-Hydroxysuccinimid in Gegenwart von Dicyclohexylcarbodiimid durch eine Verfahrensweise, die im Wesentlichen anderswo beschrieben ist (Lapidot et al., J. Lipid Res. 8, 142-145 (1967)), synthetisiert.

Die Synthese von 2-Amino-2-desoxy-1(3)-O-trityl-3(1)-O-alkyl-*sn*-glycerinen wurde in unserem Labor gemäß Bucher, Ribitsch et al. (unveröffentlicht) durchgeführt (siehe aber auch Doris Ribitsch, PhD-Thesis, Technische Universität Graz, 2002).

Rohe *Pseudomonas species*-Lipase wurde bei Nagase Biochemicals Ltd. (Japan) erworben, die reine Lipase von *Pseudomonas cepacia* (PCL) wurde von R.D. Schmid, Universität Stuttgart, Deutschland, bezogen. *Rhizopus oryzae-*Lipase wurde freundlicherweise von F. Spener und L. Haalck, Universität Münster, Deutschland, zur Verfügung gestellt.

Dichlormethan (Riedel-de Haën) wurde durch Refluxieren mit Phosphorpentoxid (Merck) getrocknet und anschließend destilliert. Andere Lösungsmittel waren von analytischer Qualität.

Eine TLC wurde auf Kieselgel 60 F₂₅₄-Aluminiumblechen (0,2 mm, Merck) durchgeführt, wobei folgende Mischungen verwendet wurden: Petroleumether/ Ether, 2:1, v/v (System 1), Chloroform/ Methanol/ Aceton, 10:0,2:0,2, v/v/v (System 2), oder Chloroform/ Methanol/ Aceton, 10:0,5:0,5, v/v/v (System 3) als Entwicklungslösungsmittel. Zur präparativen TLC-Reinigung wurden Kieselgel 60-Aluminiumbleche (0,2 mm, Merck) ohne Fluoreszenzindikator eingesetzt.

Die Verbindungen wurden unter UV-Licht (360 nm) und durch Verkohlung bei 120°C nach Besprühung mit 50%iger Schwefelsäure sichtbar gemacht. Phosphorhältige Verbindungen wurden durch Einfärbung mit Phosphormolybdänsäure (Dittmer & Lester, J. Lipid Res. 5, 126-127 (1964)) auf TLC-Platten sichtbar gemacht oder durch das Verfahren nach Broekhuyse (Biochim. Biophys. Acta 260,449-459 (1968)) in Lösungen quantitativ bestimmt.

Auf Kieselgel 60 (230-400 mesh, Merck) wurde eine kurze Säulenchromatographie durchgeführt. ¹H-NMR-Spektren wurden bei 199,97 MHz in deuterierten Lösungsmitteln aufgenommen, wobei ein Varian Gemini 200-Spektrometer eingesetzt wurde. Chemische Verschiebungen (δ) sind in ppm angegeben, und zwar bezogen auf Tetramethylsilan als Standard.

Positive Ionen-ESI-Massenspektren wurden an einer Standard-Kratos-Elektrospray-Ionenquelle, die an einem doppeltfokussierenden Kratos-Profil-HV-4-Magnetsektorgerät (Beschleunigungsspannung 2kV, *m*/*z*-Bereich 25 bis 2400 Da, Abtastgeschwindigkeit 10 s/Dec, Auflösung 1700 (10% Tal)) befestigt war, registriert. Die an die ESI-Quelle (Temperatur 50°C, Stickstoff-Gegenfluss 150 ml/min) angelegten Potentiale betrugen +5,98 kV an der Sprühkapillare, +3,12 kV am Zylinder und +2,57 kV an der Endplatte. Eine Harvard-Apparat 22-Spritzenpumpe wurde zur Abgabe eines konstanten Stroms (6 µl/min) an trockenem Methanol, enthaltend CsI (250 mg/l), eingesetzt. Lösungen der Proben (50 µM) im selben Lösungsmittel wurden über eine 100-µl Probenschleife injiziert. Die *m*/z-Werte sind für den stärksten Peak jedweder Isotopenverteilung angegeben.

### 3-(3-Perylenoyl)propansäuremethylester

Zu einer Lösung von 3-Carbomethoxypropionylchlorid (51,8 µl, 0,415 mmol) in 10 ml Dichlormethan wurden unter Rühren bei 0°C 64,7 mg (0,476 mmol) Aluminiumchlorid portionsweise hinzugefügt. Die Reaktion wurde 1 Stunde lang bei dieser Temperatur gehalten, und danach wurden portionsweise 100,0 mg (0,396 mmol) Perylen hinzugefügt. Die Mischung wurde 1 Stunde lang bei 0°C und danach über Nacht bei Raumtemperatur gehalten, auf etwa 10 g Eis gegossen und mit 1-3 Tropfen konzentrierter Salzsäure angesäuert. Das Produkt wurde mit Dichlormethan (6x 50 ml) extrahiert und mit Wasser (2x 10 ml) gewaschen. Die organischen Schichten wurden über Natriumsulfat getrocknet. Nach dem Abdampfen wurde der Rest durch eine Säulenchromatographie auf Kieselgel gereinigt, wobei mit Chloroform eluiert wurden, um das reine Produkt Perylenoylpropansäuremethylester (121,2 mg, 83,5%) zu erhalten. R_{f} 0,16 (System 1). ¹H-NMR (CDCl₃) (δ, ppm): 2,87 (t, 2H, J_{a,b}=J₂,₃ 6,52 Hz, 2-CH₂-COO-), 3,40 (t, 2H, J_{a,b}=J_{2,3} 6,51 Hz, 3-CH₂-CO-Ar), 3,77 (s, 3H, CH₃-OCO-), 7,47-7,65 (m, 3H, Ar), 7,74 (t, 2H, J 8,63 Hz, Ar), 7,95 (d, 1H, J 8,01 Hz, Ar), 8,14-8,31 (m, 3H, Ar), 8,56 (d, 1H, J 8,57 Hz, Ar).

### 4-(3-Perylenyl)butansäure

Zu einer Suspension von 3-Perylenoylpropansäuremethylester (360,2 mg, 0,983 mmol) in 2,0 ml Diethylenglycol wurden 98%iges Hydrazinhydrat (150,6 µl, 2,949 mmol) und pulverförmiges Kaliumhydroxid (275,3 mg, 4,916 mmol) hinzugefügt. Die Reaktionsmischung wurde 2 Stunden lang auf 140°C erhitzt, danach auf 190°C, und über Nacht gehalten, auf Raumtemperatur abgekühlt, mit 20 ml Wasser verdünnt, mit 0,5 ml konzentrierter Salzsäure angesäuert, das gebildete Präzipitat wurde abfiltriert, mit Wasser auf einen pH von 7,0 gewaschen, luftgetrocknet oder mit Aceton und danach mit einem Chloroform-Methanol-Gemisch (8:2 Vol.) gewaschen. Das Filtrat wurde *in vacuo* konzentriert. Das Rohprodukt wurde auf eine Kieselgelsäule aufgegeben und mit Chloroform bis 20% Methanol in Chloroform eluiert, um eine Perylenbutansäure (228,9 mg, 68,8%) zu erhalten. R_{f} 0,66 (System 3). ¹H-NMR (CDCl₃/ DMSO-d₆, 20:1) (δ, ppm): 1,92 (quin., 2H, 3-CH₂), 2,38 (t, 2H, J 7,00 Hz, 2-CH₂), 3,03 (t, 2H, J 7,00 Hz, 4-CH₂), 7,41 (d, 1H, J 7,82 Hz, Ar), 7,43-7,64 (m, 3H, Ar), 7,76 (d, 1H, J 3,42 Hz, Ar), 7,80 (d, 1H, J 3,41 Hz, Ar), 8,00 (d, 1H, J 8,49 Hz, Ar), 8,21-8,60 (m, 4H, Ar).

### 7-(3-Perylenoyl)heptansäuremethylester

Die Synthese und die Reinigung von Perylenoylheptansäuremethylester wurden erzielt wie hinsichtlich Perylenoylpropansäuremethylester beschrieben, wobei von Sebacinsäuremonomethylesterchlorid und Perylen ausgegangen wurde. Ausbeute 73,7%. R_{f} 0,23 (System 1). ¹H-NMR (CDCl₃) (δ, ppm): 1,43 (m, 4H, 4-CH₂, 5-CH₂), 1,67 (m, 2H, 3-CH₂), 1,82 (m, 2H, 6-CH₂), 2,32 (t, 2H, J_{a,b}=J_{2,3} 7,42 Hz, 2-CH₂), 3,05 (t, 2H, J_{a,b}=J_{6,7} 7,35 Hz, 7-CH₂), 3,69 (s, 3H, CH₃), 7,43-7,64 (m, 3H, Ar), 7,74 (t, 2H, J 8,26 Hz, Ar), 7,84 (d, 1H, J 7,92 Hz, Ar), 8,13-8,30 (m, 4H, Ar), 8,48 (d, 1H, J 7,54 Hz, Ar).

### 3-(3-Perylenyl)octansäure

Die Synthese und die Isolierung wurden unter identischen Bedingungen, wie hinsichtlich Perylenbutansäure beschrieben, bewerkstelligt, wobei von 7-(3-Perylenoyl)heptansäuremethylester ausgegangen wurde, und ergab 60.0% Perylenyloctansäure. R_{f} 0,63 (System 3). ¹H-NMR (CDCl₃/CD₃OD, 9:1) (δ, ppm): 1,25-1,50 (m, 6H, 3(CH₂)), 1,59 (m, 2H, 3-CH₂), 1,72 (m, 2H, 7-CH₂), 2,26 (dt, 2H, J_{a,b} 1,79 Hz, J_{2,3} 7,4.5 Hz, 2-CH₂), 2,97 (t, 2H, J_{7,8} 7,72 Hz, 8-CH₂), 7,28 (d, 1H, J 8,0 Hz, Ar), 7,43 (m, 3H, Ar), 7,59 (d, 1H, J 3,42 Hz, Ar), 7,63 (d, 1H, J 3,42 Hz, Ar), 7,83 (d, 1H, J 8,54 Hz, Ar), 8,10-8,22 (m, 4H, Ar).

### 2-(4-(3-Perylenyl)butanoyl)amino-2-desoxy-3-O-octyl-sn-glycerin (XII)

A) Zu einer Lösung von 12,0 mg (0,027 mmol) Perylenbuttersäuresuccinimidester (I) in 3 ml THF wurde 2-Amino-2-desoxy-1-O-trityl-3-O-octyl-*sn*-glycerin (V) (12,2 mg, 0,027 mmol) hinzugefügt. Die Reaktionsmischung wurde über Nacht bei Raumtemperatur gehalten und unter einem Argonstrom abgedampft. Der Rest wurde auf eine Kieselgelsäule aufgegeben und mit Chloroform eluiert. Fraktionen, die das Produkt enthielten, wurden gesammelt, und das Lösungsmittel wurde bei reduziertem Druck entfernt. Die Verbindung (XI) (20,8 mg) wurde in 98,6%iger Ausbeute erhalten. R_{f} 0,81 (System 2). Die Entfernung der Trityl-Blockierungsgruppe von 2-Perylenbutanoylamino-1-O-trityl-3-O-octyl-*sn*-glycerin (XI) wurde mit Bortrifluorid-Methanol in absolutem Dichlormethan durchgeführt, und zwar mit Hilfe eines von Hermetter et al. entwickelten Verfahrens (Chem. Phys. Lipids 50, 57-62 (1989)). Ausbeute 70.0%.
B) Die Verbindung (XII) wurde durch ein obenstehend beschriebenes analoges Verfahren in 91.0%iger Ausbeute erhalten, wobei von Perylenbutansäuresuccinimidester (I) und 2-Amino-2-desoxy-3-O-octyl-*sn*-glycerin (VI) ausgegangen wurde. Die in beiden Fällen erhaltenen Produkte waren identisch.

### 2-(4-(3-Perylenyl)butanoyl)amino-2-desoxy-1-O-octyl-sn-glycerin (XIV)

Die Verbindung (XIV) wurde unter identischen Bedingungen, wie beim Verfahren (A) für (XII) beschrieben, aus einer äquimolaren Mischung von Perylenbutansäuresuccinimidester (I) und 2-Amino-2-desoxy-1-O-octyl-3-O-trityl-*sn*-glycerin (VIII) erhalten. Die Gesamtausbeute nach zwei Stufen betrug 88,3%.

### 2-(8-(3-Perylenyl)octanoyl)amino-2-desoxy-1-O-hexadecyl-sn-glycerin (XVI)

Dieses Produkt wurde unter Anwendung derselben Verfahrensweise wie für (XII (Verfahrensweise A) synthetisiert, wobei von Perylenoctansäuresuccinimidester (II und 2-Amino-2-desoxy-1-O-hexadecyl-3-O-trityl-*sn*-glycerin (VIII) ausgegangen wurde. Ausbeute (nach zwei Stufen) 90,0%.

### 2-(3-(3-Perylenyl)octanoyl)amino-2-desoxy-3-O-hexadecyl-sn-glycerin (XVIII)

Die Verbindung (XVIII) wurde mittels derselben Verfahrensweise, wie hinsichtlich (XII) (A) beschrieben, in 67,9%iger Ausbeute hergestellt, wobei von Aminoglycerin (IX) und Perylenoctansäuresuccinimidylester (II) ausgegangen wurde.

### 2-(6-NBD-hexanoyl)amino-2-desoxy-3-O-octyl-sn-glycerin (XX)

Das Aminoglycerin (XX) wurde aus NBD-Hexansäuresuccinimidester (III) und 2-Amino-2-desoxy-1-O-trityl-3-octyl-*sn*-glycerin (V) erhalten, wie hinsichtlich (XII) (A) beschrieben. Ausbeute 86,0%. Alternativ wurde dieselbe Verbindung (XX) unter identischen Bedingungen wie bei (XII), Verfahren (B), in 100%er Ausbeute hergestellt, wobei allerdings Succinimidester (III) und 2-Amino-2-desoxy-3-O-octyl-*sn*-glycerin (VI) verwendet wurden.

### 2-(6-NBD-dodecanoyl)amino-2-desoxy-3-O-hexadecyl-sn-glycerin (XXI)

Die Synthese des langkettigen Alkylacylaminoglycerins (XXI) wurde unter identischen Bedingungen, wie hinsichtlich der Verbindung (XII) (Verfahren B) beschrieben, erzielt, wobei von NBD-Dodecansäuresuccinimidester (IV) und 2-Amino-2-desoxy-3-O-hexadecyl-*sn*-glycerin (X) ausgegangen wurde. Die Ausbeute umfasste 98,0%.

### 2-(4-(3-Perylenyl)butanoyl)amino-2-desoxy-3-O-octyl-sn-glycero-1-O-hexylphosphonat-p-nitrophenylester (XXII)

Zu einer Lösung des Glycerinderivats (XII) (20,0 mg, 0,038 mmol), 40 µl Triethylamin und einer 3,5%igen Tetrazollösung in Acetonitril (7,4 µl, 0,004 mmol) in 2 ml Dichlormethan wurde bei 5°C Hexylphosphonsäuredichlorid (12,9 µl, 0.076 mmol) hinzugefügt. Nach 1 Stunde, als die Reaktion beendet war, wurde *p*-Nitrophenol (10,6 mg, 0,076 mmol) hinzugefügt, und die Reaktionsmischung wurde über Nacht bei Raumtemperatur gehalten. Das Lösungsmittel wurde unter einem Stickstoffstrom entfernt. Das Produkt wurde im umgebenden System aus Chloroform/ Methanol/ Aceton, 10:0,2:0,2 Vol., durch präparative TLC isoliert. Ausbeute 13,1 mg (43,3%). ¹H-NMR (CDCl₃) (δ, ppm): 0,87 (t, 6H, 2CH₃), 1,05-2,00 (m, 22H, CH₂-Alkyl), 2,13 (m, 2H, 2-CH₂-Acyl), 2,30 (m, 2H, 3-CH₂-Acyl), 3,05 (m, 2H, CH₂-O-Alkyl), 3,40 (m, 4H, CH₂-O-Glycerin, 4-CH₂-Acyl), 4,25 (m, 3H, CH₂-O-P, CH-N), 6,18 (dd, 1H, NH), 7-29-7,40 (m, 3H, Ar), 7,50 (m, 3H, Ar), 7,66 (d, 1H, J.39 Hz, Ar), 7,70 (d, 1H, J 2,19 Hz, Ar), 7,89 (dd, 1H, J 3,42 Hz, J 8,06 Hz, Ar), 8,17 (m, 6H, Ar).

### 2-(4-(3-Perylenyl)butanoyl)amino-2-desoxy-1-O-octyl-sn-glycero-3-O-hexylphosphonat-p-nitrophenylester (XXVI)

Zu einer Lösung von 2-Perylenbutanoylamino-1-O-octyl-*sn*-glycerin (XIV) (8,1 mg, 0,016 mmol) und N-Methylimidazol (6,0 µl, 0,070 mmol) in 2 ml Dichlormethan wurde *n*-Hexylphosphondichlorid (10,8 µl, 0,063 mmol) hinzugefügt. Die Reaktionsmischung wurde 3 Stunden lang bei Raumtemperatur gehalten. Nach Beendigung der Reaktion wurde eine Mischung aus *p*-Nitrophenol (10,6 mg, 0,076 mmol) und N-Methylimidazol (6,0 µl, 0,070 mmol) hinzugegeben. Nach 18 Stunden wurde das Lösungsmittel unter einem Argonstrom abgedampft. Das Phosphonat (XXVI) wurde mittels TLC im System 2 gereinigt. Ausbeute 0,7 mg (5,7%).

### 2-(8-(3-Perylenyl)octanoyl)amino-2-desoxy-3-O-hexadecyl-sn-glycero-1-O-hexylphosphonat-p-nitrophenylester (XXIII)

Das Phosphonat (XXIII) wurde unter identischen Bedingungen, wie hinsichtlich (XXII) beschrieben, in 6,3%iger Ausbeute hergestellt, und ebenso unter Bedingungen wie bei (XXIV) (Ausbeute 31,6%), wobei von (XVIII) ausgegangen wurde.

### 2-(8-(3-Perylenyl)octanoyl)amino-2-desoxy-1-O-hexadecyl-sn-glycero-3-O-hexylphosphonat-p-nitrophenylester (XXVII)

Die Synthese und die Isolierung wurden gemäß der hinsichtlich (XXII) beschriebenen Verfahrensweise bewerkstelligt, wobei vom Aminoglycerin-Analog (XVI) ausgegangen wurde. Ausbeute 26,1%.

### 2-(NBD-Hexanoyl)amino-2-desoxy-3-O-octyl-sn-glycero-1-O-hexylphosphonat-p-nitrophenylester (XXIV)

Das Produkt (XXIV) wurde unter identischen Bedingungen, wie hinsichtlich (XXII) beschrieben, aus fluoreszenzmarkiertem Glycerin (XX) in 27,8%iger Ausbeute erhalten.

### 2-(NBD-Dodecanoyl)amino-2-desoxy-3-O-hexadecyl-sn-glycero-1-O-hexylphosphonat-p-nitrophenylester (XXXV)

Die Verbindung (XXI) wurde phosphoryliert wie hinsichtlich der Herstellung von Phosphonat (XXII) beschrieben, wobei als Ausgangsmaterial Acylamino-O-alkylglycerin (XXI) verwendet wurde. Die Ausbeute umfasste 4,4%.

### ((6-(7-Nitrobenz-2-oxa-1,3-diazol-4-yl)amino)hexanoyl)aminoethanol (XXX)

Eine Mischung aus NBD-Hexansäuresuccinimidylester (12,5 mg, 0,032 mmol) und 2-Aminoethanol (2 µl, etwa 0,032 mmol) in 2 ml THF wurde 30 Minuten lang stehen gelassen. Nachdem die TLC anzeigte, dass die Reaktion beendet war, wurde das Lösungsmittel unter einem Argonstrom entfernt. Der Rest wurde in einem Chloroform/ Methanol/ WasserGemisch (65:25:4 Vol.) (5 ml) aufgelöst, und 1 g Dowex 50Wx8-Harz (H⁺-Form) wurde hinzugefügt.. Nach 30-minütigem Rühren wurde das aufgelöste Produkt (XXX) filtriert und das Harz mit demselben Lösungsmittelsystem gewaschen (3x 5 ml). Das Filtrat wurden bei reduziertem Druck abgedampft. Eine TLC-Analyse zeigte eine einzige fluoreszierende Stelle mit R_{f} 0,69 und kein Aminoethanol nach der Besprühung mit Ninhydrin. Ausbeute 10,6 mg (98,3%).

### O-(((6-(7-Nitrobenz-2-oxa-1,3-diazol-4-yl)amino)hexanoyl)aminoethyl-O-(p-nitrophenyl)-n-hexylphosphonat (XXXI)

Unter Anwendung des hinsichtlich Phosphonat (XXII) beschriebenen Verfahrens wurde NBD-markiertes Hexylphosphonat erhalten, wobei vom entsprechenden Alkohol (XXX) ausgegangen wurde. Die Ausbeute umfasste 11,1%.

### Bestimmung der Lipaseaktivität

Die Hemmung der Lipaseaktivität mittels wasserunlöslicher Phosphonate wurde in Triton X-100-Mizellen (2 mM Endkonzentration) durchgeführt und durch ein kontinuierliches Fluoreszenzverfahren gemäß Duque et al. gemessen (J. Lipid Res. 37, 868-876 (1996)), wobei 3-O-Hexadecyl-2-pyrendecanoyl-1-trinitrophenylaminododecanoyl-*sn*-glycerin als Substrat eingesetzt wurde. Die Lipaseaktivität wurde bei 30°C durch das Dequenchen der Pyren-Fluoreszenz bei 378 nm (Anregung bei 342 nm, Schlitzbreite jeweils 5,0 nm) bestimmt (der anfängliche lineare Anstieg der Fluoreszenzintensität nach Zugabe der Lipase wurde zur Messung der Enzymaktivität eingesetzt). Die Messungen wurden in Ethanol-Wasser (1:3 Vol.) als Lösungsmittel durchgeführt.

### Inhibitionsversuche

Speziell für eine Membranuntersuchung gereinigtes Triton X-100 (Hofmann La Roche) (10 µl einer 100 mM-Stammlösung in Chloroform) und das entsprechende Volumen einer Phosphonatlösung in Chloroform wurden vermischt, und das Lösungsmittel wurde unter einem Stickstoffstrom entfernt. Der Rest wurde durch kräftiges Verwirbeln in einem 100 µM-Tris-HCl-Puffer (pH 7,40 bei 37°C) dispergiert, gefolgt von der Zugabe eines Aliquots Lipaselösung, wodurch sich ein Gesamtvolumen von 500 µl ergab. Bei 4°C wurden Inkubationen durchgeführt. Der nach 16 Stunden in der Reaktionsmischung auftretende prozentuale Anteil an Inhibition wurde durch Messung der restlichen Lipaseaktivität von entsprechenden Aliquoten (3 µl) bestimmt, wie obenstehend angegeben. Gleichzeitig wurden unter identischen Bedingungen, jedoch in Abwesenheit von Inhibitoren, Kontrollen durchgeführt.

### Spektroskopische Analysen

Ein molarer Extinktionskoeffizient von 22000 M⁻¹cm⁻¹ bei 466 nm in Methanol oder von 29000 M⁻¹ cm⁻¹ bei 442 nm in Ethanol für mit NBD bzw. Perylen markierte Phosphonatinhibitoren wurde zur Bestimmung der Konzentration der fluoreszierenden Phosphonate eingesetzt (die Messungen wurden mit einem U-3210-Spektrophotometer von Hitachi durchgeführt) (Haugland, Handbook of Fluorescent Probes, Molecular Probes, S. 293 (1996)).

### Ergebnisse:

### Chemische Synthesen

Acht neuartige, mit Perylenbutan-, Perylenoctan-, NBD-Hexan- oder NBD-Dodecansäure fluoreszenzmarkierte Phosphonsäureester wurden synthetisiert. Perylen- und NBD-Markierungen wurden aufgrund der im Vergleich zu den zuvor synthetisierten und charakterisierten, pyrenhaltigen Inhibitoren längeren Wellenlängenabsorption und -emission ausgewählt (Zandonella et al., siehe oben).
Zur Herstellung von Perylen-markierten Lipaseinhibitoren wurden die entsprechenden fluoreszenzmarkierten Perylenfettsäuren benötigt. Die Friedel-Crafts-Acylierung von Perylen mit geeigneten Dicarbonsäuremonomethylesterchloriden ergab einen Perylenoylpropionsäuremethylester und Perylenoylheptansäuremethylester. Es ist bekannt, dass Perylen unter diesen Reaktionsbedingungen nur an der C-3-Position acyliert wird (Zinke et al., Ber. B75, 1042-1048 (1940)). Die ¹H-NMR-Spektren der synthetisierten Perylenoylpropion- und Perylenoylheptansäuren (Protone der Alkyl- und aromatischen Reste) bestätigten die angedeuteten Strukturen der Acylierungsprodukte. Eine Kizhner-Wolf-Reduktion der Ketogruppen und die gleichzeitige Freigabe der Methylschutzgruppen ergab Perylenbutan- bzw. Perylenoctansäuren. Die Protonen-NMR-Spektren stimmten mit den zugeschriebenen Strukturen überein.

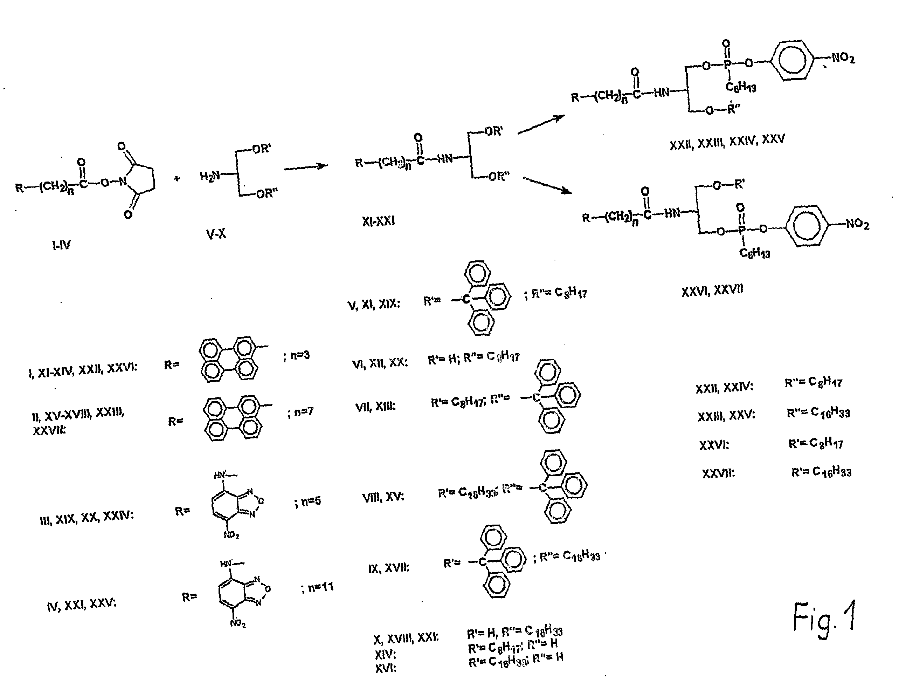

Die zu den fluoreszenzmarkierten Triacylglycerin-Analoga führenden Syntheserouten sind in Fig. 1 dargelegt. Die jeweiligen Verbindungen sind Alkylacyl(amino)desoxy-glycero-phosphonsäuren und Alkyloxyphosphonsäurederivate, an deren Synthese drei Hauptschritte von entscheidender Bedeutung beteiligt sind, und zwar: 1) Anbringung einer fluoreszierenden Acylkette an 1(3)-Alkyl-2-aminodesoxy-3(1)-trityl-*sn*-glycerin, 2) Detritylierung und 3) Phosphorylierung der sich ergebenden Alkylacyl(amino)desoxy-glycerine.

Im Prinzip kann die Acylierung einer Aminogruppe unter Venvendung eines Säureanhydrids (Singh et al., J. Carbohydr. Chem. 8, 199-216 (1989)), von Acyl-Haliden (Zimmerman et al., j. Carbohydr. Chem. 7, 435-452 (1988); Dijkman et al., Biochim. Biophys. Acta 1043, 67-74. (1990)), von gemischtem Carbodiimid (Hammarstroem, J. Lipid Res. 12, 760-765 (1971)) oder von gemischten Anhydriden, gebildet aus Ethylchlorformat und einer Fettsäure (Acquotti et al., Chem. Phys. Lipids 40, 71-86 (1986)), durchgeführt werden.

Diese Verfahrensweisen haben mehrere Nachteile, insbesondere da sie einen großen molaren Überschuss an Acylierungsreagenzien und lange Reaktionszeiten benötigen. Die Verwendung von Acylchloriden war aufgrund der möglichen Modifikation von Fettsäurederivaten während der Acylierung in unserem Fall nicht angebracht. Aktivierte Fettsäureester (beispielsweise *p*-Nitrophenylester) wurden ebenfalls erfolgreich angewandt, z.B. bei der Sphingolipidsynthese (Tkaczuk et al., J. Org. Chem. 46, 4393-4398 (1981); Groenberg et al., Biochemistry 30, 10746-10754 (1991); Kann et al., Biochemistry 30, 7759-7766 (1991); Shibuya et al., Chem. Pharm. Bull. 40, 1154-1165 (1992)).

Eine Acylierung mit den *p*-Nitrophenylestern von Perylenbutter- und Perylenoctansäuren kann jedoch wahrscheinlich aufgrund der großen Nähe zwischen einem sperrigen Fluorophor und der Estergruppe nur geringe Ausbeuten an Acylierungsprodukten erbringen (Oskolkova et al., Chem. Phys. Lipids 99, 73-86 (1999)). Somit verwendeten wir N-Hydroxysuccinimidester von fluoreszenzmarkierten Fettsäuren, um in Gegenwart von freien Hydroxygruppen Aminogruppen in großen Ausbeuten selektiv zu acylieren (Julina et al., Helv. Chim. Acta 69, 368-373 (1986)). Bei der vorliegenden Erfindung erbrachte dieses einfache Verfahren große Ausbeuten an Acylierungsprodukten (z.B., 91.0%ige Ausbeute an (XII) bei Acylierung von 2-Amino-2-desoxy-3-O-octyl-*sn*-glycerin (VI) mit Perylenbutansäuresuccinimidester).

Die schützende Tritylgruppe der markierten Alkylacylaminodesoxytritylglycerine wurde unter Standardbedingungen entfernt, wie hinsichtlich der Synthese von Diradylglycerinen beschrieben (Hermetter et al., / 989, siehe oben), wobei Bortrifluorid-Methanol eingesetzt wurde. Die erhaltenen fluoreszierenden Alkylacylaminodesoxyglycerine (XI-XVIII) zeigten Fluoreszenzspektren, die für Perylen-markierte Verbindungen typisch waren (Johanson et al., J. Am. Chem. Soc. 109, 7374-7381 (1987)).

Zwei weitere Probleme werden bei der Synthese von fluoreszierenden Phosphonatinhibitoren angetroffen. Die Reaktionsausbeuten einer Phosphorylierung von Glycerolipiden tendieren dazu, gering zu sein. Zweitens sind die fluoreszenzmarkierten Fettsäuren und folglich die fluoreszenzmarkierten Glycerolipid-Zwischenverbindungen kostspielig, und dies ist ein besonderer Grund, weshalb die Ausbeuten so groß wie möglich sein sollten.

Die Diradylglycerine wurden in Gegenwart von N-Methylimidazol als Base mit Alkylphosphonsäuredichlorid umgesetzt, gefolgt von einer Substitution des zweiten Chloratoms am Phosphor durch *p*-Nitrophenol. Obwohl diese beiden Schritte nacheinander in einer Einstufenreaktion durchgeführt wurden, waren die Produktausbeuten relativ gering (5,7% bei 2-Desoxy-2-(4-perylenylbutanoyl)amino-1-O-octyl-3-O-hexylphosphonat (XXVI) oder 6,3% bei 2-Desoxy-2-(8-gerylenyloctanoyl)amino-3-O-hexadecyl-1-O-hexylphosphonat (XXIII)), und zwar am wahrscheinlichsten aufgrund der großen Nähe zwischen der Amidogruppe und dem Phosphorsäurerest, was in der Folge zu einem intramolekularen cyclischen Nebenprodukt (Oxazaphospholan) in beträchtlichen Mengen führen kann.

Solche Verbindungen können in der Tat in Gegenwart von Phosphoroxytrichlorid aus 2-Desoxy-2-aminophosphocholin hergestellt werden (Deigner et al., Chem. Phys. Lipids 61, 199-208 (1992)). Um die Bildung solcher Verbindungen zu vermeiden, wandten wir zur Synthese von Phosphonatestern eine Abwandlung des bei Zhao et al. (Tetrahedron Lett. 49, 363-368 (1993)) beschriebenen Phosphorylierungsverfahrens an. Diese Methode setzte Tetrazol als Katalysator ein und erwies sich bereits bei der Synthese von Phosphonaten aus sterisch gehinderten Alkoholen, wie z.B. Menthol und Testosteron (Zhao et al., siehe oben), oder bei der Synthese von Organophosphonatestem (Rotticci et al., siehe oben) als erfolgreich.

In unserem Fall wurden die Produktausbeuten beträchtlich verbessert, wenn als Katalysator Tetrazol eingesetzt wurde (z.B. betrugen die Ausbeuten an 2-Desoxy-2-(8-perylen-octanoyl)amino-3-O-hexadecyl-1-O-hexylphosphonat (XXIII) bei Fehlen von Tetrazol 6,3% und in dessen Gegenwart 31,6% (Tabelle 1)). Im Fall der anderen Glycerolipidphosphonat-Analoga lagen die Ausbeuten bei einer Synthetisierung durch die Tetrazol-katalysierte Reaktion bei über 25%.

**Tabelle 1. Vergleich der chromatographischen Charakteristika von synthetisierten fluoreszierenden Phosphonaten und deren Ausbeuten nach der Phosphorylierung.**

| **Verbindungen** | **Nummer** | **Chromatographisches Verhalten** R_{f} | **Phosphorylierungsausbeute,** % | |
|---|---|---|---|---|
| | | | **ohne Katalysator** | mit **Tetrazol als Katalysator** |
| 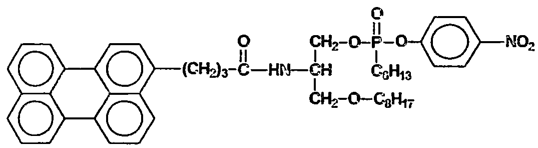 | **XXII** | 0,33^{a} | n.d. | 43,3 |
| 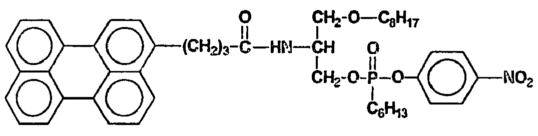 | **XXVI** | 0,33^{a} | 5,7 | n.d. |
| 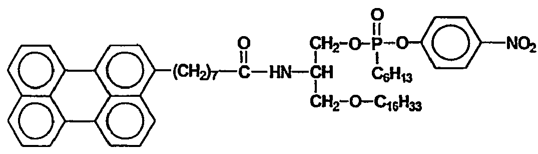 | **XXIII** | 0,46^{a} | 6,3 | 31,6 |
| 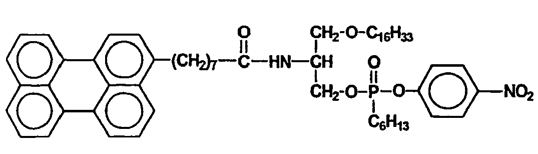 | **XXVII** | 0,46^{a} | n.d. | 26,1 |
| 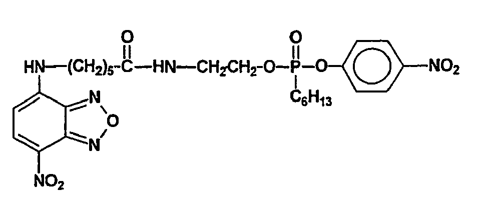 | **XXXI** | 0,04^{b} | n.d. | 11,1 |
| 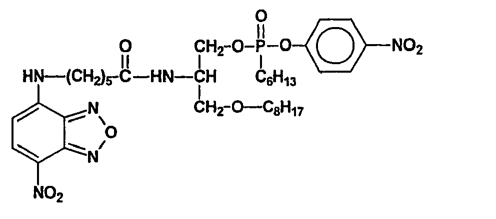 | **XXIV** | 0,16^{b} | n.d. | 27,8 |
| 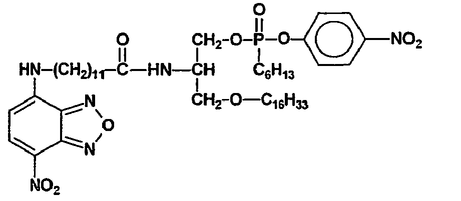 | **XXV** | 0,55^{b} | n.d. | 4,4 |

| | | | | |
|---|---|---|---|---|
| n.d. = nicht detektierbar ^{a} System für TLC: Chloroform/ Methanol/ Aceton, 10:0,2:0,2 Vol.; ^{b} In Chloroform/ Ethanol/ Aceton, 10:0,5:0,5 Vol. | | | | |

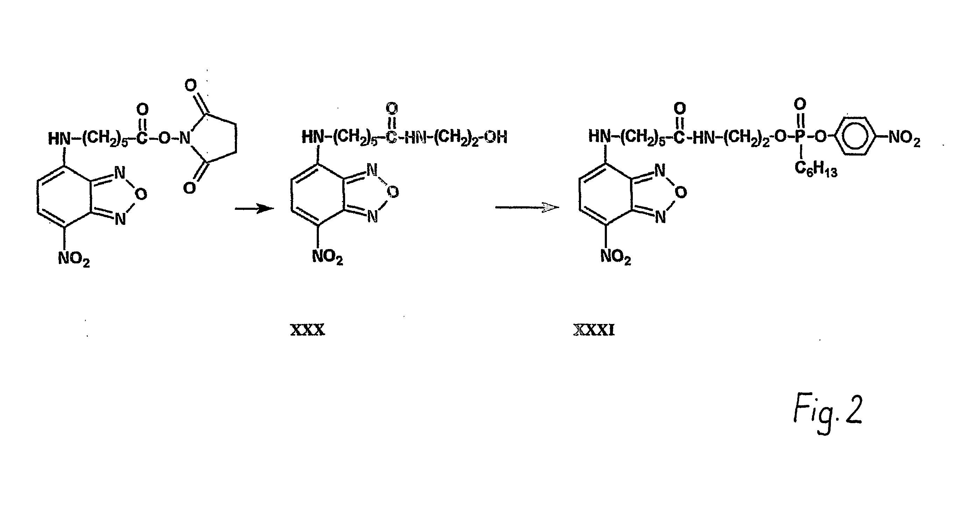

Als zweite Klasse von Lipaseinhibitoren wurden Phosphonate, die anstelle von Glycerolipiden einkettige Alkoxygruppen enthielten, wie folgt hergestellt (Fig. 2). Typischerweise wurde NBD-Hexansäuresuccinimidester mit Aminoethanol umgesetzt, wodurch das Derivat (XXX) hervorgebracht wurde. Die Phosphorylierung letzterer Verbindung ergab das Phosphonat (XXXI) in 11.1%iger Ausbeute.

Die Protonensignale im ¹H-NMR-Spektrum von Phosphonat (XXII) stimmten mit der angedeuteten Molekularstruktur überein. Da in dieser Studie die Phosphorylierungsverfahren für alle Glycerolipide identisch waren, wird nur ein Protonen-NMR-Spektrum für letztere Verbindung vorgelegt. Die UV- und Fluoreszenzspektren aller Perylen-markierten Organophosphorverbindungen zeigten identische λₘₐₓ, die mit ihren vermuteten chemischen Strukturen übereinstimmen.

Die Perylen- (XXII, XXIII, XXVI, XXVII) und NBD-Phosphonate (XXIV, XXV, XXXI) hatten in Ethanol Fluoreszenzmaxima bei 448 nm und 533 nm, welche für NBD-hältige Derivate jeweils typisch waren. Das Verhältnis zwischen Fluoreszenzmarkierung und Phosphorgehalt war bei allen Phosphonaten etwa 1 bis 1,15, was zusätzlich die Strukturen der erhaltenen Verbindungen bestätigte. Überdies wurden in den ESI-Massenspektren der Verbindungen (XXII, XXVI, XXIII, XXVII, XXXI, XXIV, XXV) nur Signale, die mit ihren Molekularstrukturen übereinstimmten, beobachtet (Tabelle **2).**

**Tabelle 2. Ergebnisse einer Massenspektrometrieanalyse von Organophosphonaten.**

| **Verbindung Nummer** | **Formel** | **Molekülmasse** | beobachtete Ionen | **m/Zₜₕₑₒᵣ** | **m/z_{beobachtet}** |
|---|---|---|---|---|---|
| **XXII** | C₄₇H₅₇N₂O₇P | 792,39 | [C₄₇H₅₇N₂O₇P•Cs]⁺ | 925,30 | 925,4 |
| **XXVI** | C₄₇H₅₇N₂O₇P | 792,39 | [C₄₇H₅₇N₂O₇P•Cs]⁺ | 925,30 | 925,3 |
| **XXIII** | C₅₉H₈₁N₂O₇P | 960,58 | [C₅₉H₈₁N₂O₇P•Cs]⁺ | 1093,48 | 1093,4 |
| **XXVII** | C₅₉H₈₁N₂O₇P | 960,58 | [C₅₉H₈₁N₂O₇P•Cs]⁺ | 1093,48 | 1093,4 |
| **XXXI** | C₂₆H₃₅N₆O₉P | 606,22 | [C₂₆H₃₅N₆O₉P•Cs]⁺ | 739,13 | 739,3 |
| **XXIV** | C₃₅H₅₃N₆O₁₀P | 748,36 | [C₃₅H₅₃N₆O₁₀P•Cs]⁺ | 881,26 | 881,3 |
| **XXV** | C₄₉H₈₁N₆O₁₀P | 944,58 | [C₄₉H₈₁N₆O₁₀P•Cs]⁺ | 1077,48 | 1077,3 |

Die Massenspektren wurden wie in "Materialien und Verfahren" beschrieben aufgenommen.

### Wechselwirkung zwischen Inhibitoren und fettspaltenden Enzymen

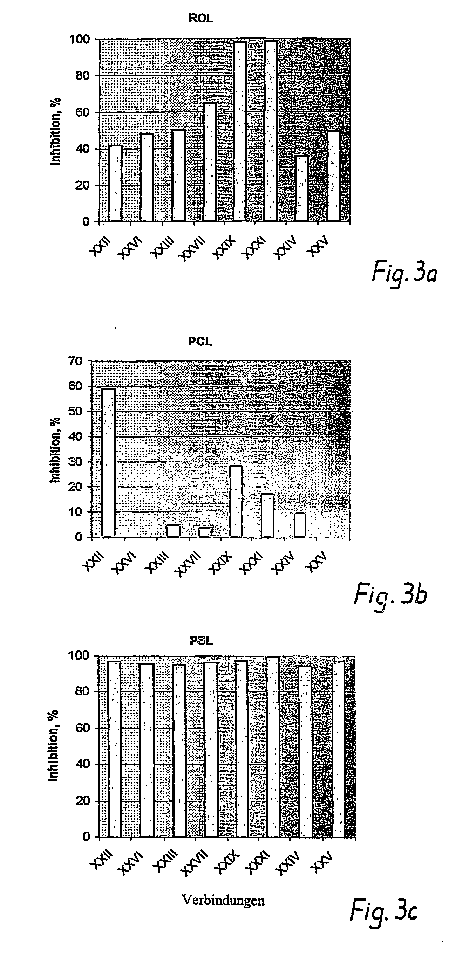

Die neuartigen Verbindungen wurden hinsichtlich ihrer Fähigkeit, drei ausgewählte mikrobielle Lipasen mit unterschiedlichen Substratpräferenzen zu hemmen, untersucht. Die Lipase von *Rhizopus oryzae* wurde bei 4°C 16 Stunden lang in einem 100 µM-Tris-HCl-Puffer durch die Perylen- und NBD-Inhibitoren bei Inhibitor- und Lipasekonzentrationen von 1,0 mM bzw. 0,1 mM wirksam deaktiviert (Fig. 3a). Es ist erwähnenswert, dass die langkettigen Inhibitoren (XXIII) und (XXVII) im Vergleich zu den entsprechenden kurzkettigen Verbindungen (XXII) und (XXVI) etwas aktiver waren. Das langkettige NBD-Dodecanoylamino-*sn*-1-phosphonat (XXV) war etwas wirksamer als sein kurzkettiges Gegenstück (XXIV). Dies entspricht der Annahme, dass Lipasen als Substrate langkettige Glycerinester vorziehen, und offensichtlich ebensolche Inhibitoren.
Das zweikettige Phosphonat (XXII) war ein viel wirksamerer Inhibitor für die Lipase von *Pseudomonas cepacia* als die anderen Inhibitoren, die verwendet wurden (Fig. 3b). Im Gegensatz dazu wurde die *Pseudomonas species*-Lipase durch alle synthetisierten Organophosphonate quantitativ deaktiviert (Fig. 3c).

Lipasen können im Umkreis ihrer aktiven Stelle und in dieser sehr unterschiedliche sterische Einschränkungen und folglich sehr unterschiedliche Substrat- und Inhibitorpräferenzen aufweisen (Pleiss et al., Chem. Phys. Lipids 93, 67-80 (1998)). Die Lipasen ROL, PCL und PSL, die für die Inhibierungsversuche mit Organophosphonaten ausgewählt wurden, sind typische Beispiele für diese strukturelle Vielfalt unter hoch homologen Enzymen. Demgemäß weisen sie unterschiedliche Reaktivitätsmuster auf, und zwar nicht nur in Hinblick auf Lipidsubstrate, sondern auch auf strukturell verwandte Inhibitoren, wie hier gezeigt.

Als Alternative zu den in der Industrie eingesetzten wasserlöslichen Lipasemonomeren wurden zur Steigerung der Enzymaktivität (Zelinski et al., Angew. Chem. 109, 746-748 (1997)) und der Stabilität in organischen Lösungsmitteln (Persichetti et al., 1995) vernetzte Enzymkristalle (CLECs) (Khalaf et al., J. Am. Chem. Soc. 118, 5494-5495 (1996); Lalonde et al., J. Am. Chem. Soc. 117, 6845-6852 (1995); Clair et al., J. Am. Chem. Soc. 114, 7314-7316 (1992)) eingeführt. Dies macht sie zu nützlichen Katalysatoren, welche leicht von der Reaktionsmischung zu trennen sind und nach anschließender Filtration und Waschung wiederholt verwendet werden können. Die erfindungsgemäßen Inhibitoren könnten auch bei der Charakterisierung der funktionellen Qualität dieser Systeme von Nutzen sein, die ansonsten schwierig zu bestimmen ist.

## Patentansprüche

1. Verbindung mit der allgemeinen Formel I 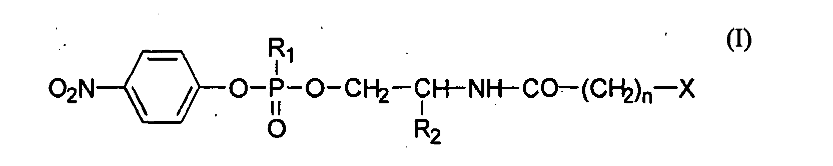 wobei X einen optisch detektierbaren Rest darstellt, n eine ganze Zahl ist, wobei 1 ≤ n ≤ 20, R₁ eine unverzweigte oder verzweigte Alkylgruppe mit 1 bis 20 Kohlenstoffatomen und R₂ Wasserstoff oder eine -CH₂-O-R₃-Gruppe ist, wobei R₃ dieselbe Bedeutung hat wie R₁.

2. Verbindung gemäß Anspruch 1, wobei n eine ganze Zahl ist, wobei 3 ≤ n ≤ 11.

3. Verbindung gemäß Anspruch 1 oder 2, wobei R₁ Hexyl und R₂ eine -CH₂-O-R₃-Gruppe ist, wobei R₃ Octyl oder Hexadecyl ist.

4. Verbindung gemäß Anspruch 1 oder 2, wobei R₁ Methyl und R₂ eine -CH₂-O-R₃-Gruppe ist, wobei R₃ Octyl oder Hexadecyl ist.

5. Verbindung gemäß Anspruch 1 oder 2, wobei R₁ Butyl und R₂ eine -CH₂-O-R₃-Gruppe ist, wobei R₃ Octyl oder Hexadecyl ist.

6. Verbindung gemäß einem der Ansprüche 1 bis 5, wobei der optisch detektierbare Rest ein Fluorophor ist.

7. Verbindung gemäß Anspruch 6, wobei der Fluorophor eine Perylen-, Pyren- oder Nitrobenzoxadiazol-Gruppe ist.

8. Verbindung gemäß einem der Ansprüche 1 bis 7 zur Verwendung bei der Hemmung fettspaltender Enzyme.

9. Verbindung gemäß einem der Ansprüche 1 bis 7 zur Verwendung bei der Bestimmung und/oder Unterscheidung fettspaltender Enzyme in biologischen Proben.

## Claims

1. A compound having general formula I 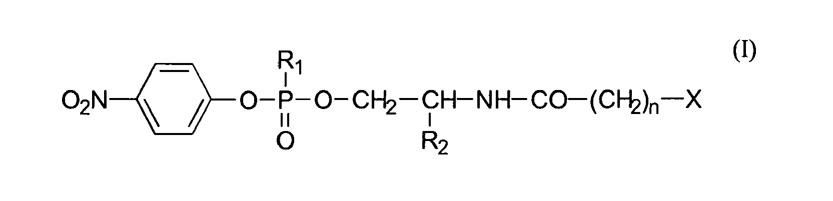 in which X represents an optically detectable moiety, n is an integer with 1 ≤ n ≤ 20, R₁ is an unbranched or branched alkyl group having 1 to 20 carbon atoms, and R₂ is hydrogen or -CH₂-O-R₃ group, wherein R₃ has the same meaning as R₁.

2. A compound according to claim 1, wherein n is an integer with 3 ≤ n ≤ 11.

3. A compound according to claim 1 or 2, wherein R₁ is hexyl and R₂ is -CH₂-O-R₃ group with R₃ being octyl or hexadecyl.

4. A compound according to claim 1 or 2, wherein R₁ is methyl and R₂ is -CH₂-O-R₃ group with R₃ being octyl or hexadecyl.

5. A compound according to claim 1 or 2, wherein R₁ is butyl and R₂ is -CH₂-O-R₃ group with R₃ being octyl or hexadecyl.

6. A compound according to any of claims 1 to 5, wherein the optically detectable moiety is a fluorophore.

7. A compound according to claim 6, wherein the fluorophore is a perylene, pyrene or nitrobenzoxadiazole group.

8. A compound according to any of claims 1 to 7 for use in the inhibition of lipolytic enzymes.

9. A compound according to any of claims 1 to 7 for use in the determination and/or discrimination of lipolytic enzymes in biological samples.

## Revendications

1. Composé de formule générale 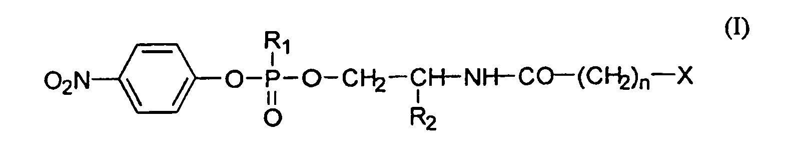 dans laquelle X représente un radical optiquement détectable, n est un nombre entier, où 1 ≤ n ≤ 20, R₁ représente un groupe alkyle non-ramifié ou ramifié ayant de 1 à 20 atomes de carbone et R₂ un atome d'hydrogène ou un groupe -CH₂-O-R₃, R₃ ayant la même signification que R₁.

2. Composé selon la revendication 1, dans lequel n est un nombre entier, où 3 ≤ n ≤ 11.

3. Composé selon la revendication 1 ou 2, dans lequel R₁ est un groupe hexyle et R₂ un groupe -CH₂-O-R₃, R₃ étant un groupe octyle ou hexadécyle.

4. Composé selon la revendication 1 ou 2, dans lequel R₁ est un groupe méthyle et R₂ un groupe -CH₂-O-R₃, R₃ étant un groupe octyle ou hexadécyle.

5. Composé selon la revendication 1 ou 2, dans lequel R₁ est un groupe butyle et R₂ un groupe -CH₂-O-R₃, R₃ étant un groupe octyle ou hexadécyle.

6. Composé selon l'une quelconque des revendications 1 à 5, dans lequel le radical optiquement détectable est un fluorophore.

7. Composé selon la revendication 6, dans lequel le fluorophore est un groupe perylène, pyrène ou nitrobenzoxadiazole.

8. Composé selon l'une quelconque des revendications 1 à 7, destiné à une utilisation dans l'inhibition d'enzymes clivant les lipides.

9. Composé selon l'une quelconque des revendications 1 à 7, destiné à une utilisation dans la détermination et/ou la différenciation d'enzymes clivant les lipides dans des échantillons biologiques.
